# EUROPEAN PATENT APPLICATION

(11) **EP 4 725 411 A1**
(43) Date of publication of application: **15.04.2026**
(21) Application number: 24206551.4
(22) Date of filing: 14.10.2024
(51) Int. Cl.: A61B 5/11, A61B 5/296, A61B 5/389, A61B 5/00

(54) **ELECTROMYOGRAPHY MEASUREMENTS**

(71) Applicant: Vrije Universiteit Brussel, 1050 Brussel (BE); Imec VZW, 3001 Leuven (BE)
(72) Inventor: LANGLOIS, Kevin, 1030 Schaarbeek (BE); DE WINTER, Joris, 2610 Wilrijk (BE); VANDERBORGHT, Bram, 1560 Hoeilaart (BE)
(74) Representative: Winger

(57) **Abstract**

A system (100) for performing electromyography (EMG) measurements, the system comprising an EMG unit (110) for collecting electromyography data of a user, the EMG unit (110) comprising a plurality of EMG electrodes (120), one or more other sensors (130) for capturing sensor data related to a motion and/or activity of the user, a sensor data processing unit (140) being programmed for processing the captured sensor data using a muscle activity expectation model (142) for identifying specific muscles used during the motion and/or activity of the user, and the system being adapted for selecting a subset of EMG electrodes from the plurality of EMG electrodes (120), said selecting being based on the identified specific muscles.

## Description

### Field of the Invention

The present invention relates to the field of wearable biomechanical monitoring systems, and more specifically to devices for performing electromyography and movement analysis.

### Background of the Invention

Athlete monitoring during physical activity has become an integral component of sports science, contributing to injury prevention, performance optimization, and effective rehabilitation. Measuring muscle activity and skeletal movement provides valuable insights that assist medical professionals and coaches in assessing muscle function, identifying movement inefficiencies, and tracking recovery progress.

However, accurately capturing muscle activity and movement in real time on the field presents several challenges. Devices designed for laboratory environments often lack portability and practicality for on-field use. While portable equipment exists, it may offer limited capabilities or reduced accuracy compared to laboratory-grade systems.

The use of measurement equipment during athletic activities can also interfere with an athlete's natural movement or performance. Wearable sensors, even when intended for mobility, might cause discomfort or hinder motion, leading to reluctance among athletes to use such devices during training or competition.

Real-time data collection generates substantial amounts of information that can be complex to process and interpret immediately. Managing this data, especially without specialized training, can be challenging for users who require straightforward analysis without extensive intervention. Additionally, synchronizing data from multiple sources, such as muscle activity monitors and motion sensors, adds to the complexity and necessitates sophisticated systems.

Cost considerations further impact the adoption of high-quality, real-time measurement systems. The expense of advanced equipment may be prohibitive for smaller teams or organizations with budget constraints, limiting access to comprehensive monitoring solutions.

Given these challenges, there is an ongoing need for advancements in the field to address at least some of these issues. Enhancing the ability to monitor muscle activity and movement effectively and unobtrusively during athletic performance remains a significant area for development.

### Summary of the Invention

It is an object of embodiments of the present invention to provide a system and method for performing electromyography measurements that enable accurate and reliable muscle activity monitoring. This objective is accomplished by the aspects of the present invention.

In the first aspect, the present invention relates to a system for performing electromyography (EMG) measurements, the system comprising:
- an EMG unit for collecting electromyography data of a user, the EMG unit comprising a plurality of EMG electrodes,
- one or more other sensors for capturing sensor data related to a motion and/or activity of the user,
- a sensor data processing unit being programmed for processing the captured sensor data using a muscle activity expectation model for identifying specific muscles used during the motion and/or activity of the user, and
for the system being adapted for selecting a subset of EMG electrodes from the plurality of EMG electrodes, said selecting being based on the identified specific muscles.

The system may comprise for example an EMG electrode selecting unit for performing said selecting the subset.

The system thus may be adapted for identifying a good, e.g. optimal, electrodeset for each muscle during the motion or activity. Since the electrodes can shift over time, dynamic selection of the subset of electrodes may be performed.

This allows accurate EMG measurements to be performed by selecting and using only a relevant subset of the EMG electrodes.

In embodiments, the system may be configured for performing said capturing sensor data and said processing of the captured sensor data dynamically over time and for dynamically selecting the subset of EMG electrodes based thereon. This enables dynamically switching between subsets of EMG electrodes based on the real-time output of the muscle activity expectation model, optimizing the reliability of measurements for various individual muscles. In embodiments, the sensor data processing unit may be programmed for classifying a motion and/or activity of the user based on the captured sensor data and/or for segmentation of the captured sensor data for identifying starting times and/or ending times of a motion and/or activity of the user. This multi-modal approach allows correlating a classified motion to specific muscle activities.

In embodiments, said identifying specific muscles may be based on the classification of the motion and/or activity of the user.

In embodiments, the system may furthermore comprise an impedance detection unit configured for measuring an impedance of at least the subset of EMG electrodes. This allows eliminating electrodes exhibiting high impedance from those used for capturing data, enhancing measurement quality.

In embodiments, the EMG electrode selecting unit may furthermore be configured for removing electrodes from the subset of EMG electrodes to be used for capturing electromyography data based on one or more of at least a high impedance value and/or a degree of correlation between a measured EMG signal and a reference EMG signal.

A degree of correlation between a measured EMG signal and a reference EMG signal may refer to the extent to which the collected EMG signal matches an expected signal pattern, which may indicate proper electrode function and muscle activation. An example is comparing the frequency content of the measured signal to that of a known muscle activation pattern. A reference EMG signal may refer to a standard or expected EMG signal used for comparison purposes, which may be derived from previous measurements, stored data, or literature. Examples include baseline muscle activation signals during a known activity.

In embodiments, the one or more other sensors may comprise one or more of Inertial Measurement Units (IMUs), stretch sensors, force sensors, pressure sensors, camera systems.

In embodiments, the muscle activity expectation model may be based on one or more of a machine learning technique, statistical data, time-series data, pattern recognition techniques, domain-specific knowledge, fuzzy logic, time-frequency data, to dynamically identify specific muscles used during the motions and/or activities of the user.

In embodiments, the system may be a wearable system. This allows integrating the sensors and electrodes into a piece of clothing like trousers or a shirt.

In embodiments, the system may be configured for performing electromyography measurements for motion or activity of humans in the field of sports science, healthcare or ergonomics or for performing electromyography measurements for motion or activity of animals. The muscle activity expectation model can be tailored to the specific application domain.

In the second aspect, the present invention relates to a method for performing electromyography (EMG) measurements, the method comprising:
- receiving sensor data related to a motion and/or activity of a user,
- processing the received sensor data using a muscle activity expectation model for identifying specific muscles used during the motion and/or activity of the user, and selecting a subset of EMG electrodes from a plurality of EMG electrodes, said selecting being based on the identified specific muscles, and
- using said subset of EMG electrodes for collecting electromyography data of a user.

In embodiments, the method may comprise performing said capturing sensor data and said processing of the captured sensor data dynamically over time, for dynamically selecting the subset of EMG electrodes based thereon and dynamically collecting electromyography data using said dynamically selected subset of EMG electrodes.

In embodiments, the method may furthermore comprise measuring an impedance of the EMG electrodes and removing EMG electrodes with high impedance from the subset of EMG electrodes used for collecting electromyography data or with an impedance differing substantially from a reference impedance for the EMG electrode.

In the third aspect, the present invention relates to a computer program product for, when executed on a processor, assisting in performing electromyography (EMG) measurements, by
- receiving sensor data related to a motion and/or activity of a user,
- processing the received sensor data using a muscle activity expectation model for identifying specific muscles used during the motion and/or activity of the user,
- selecting a subset of EMG electrodes from a plurality of EMG electrodes, said selecting being based on the identified specific muscles, and
- using said subset of EMG electrodes for collecting electromyography data of a user. Selecting the subset of EMG electrodes may be performed for identifying a good, e.g. optimal, electrodeset for each muscle during the motion or activity.

It is an advantage of embodiments of the present invention that seamless integration of sensors into apparel is possible, eliminating the need for external devices or obstructive equipment and improving user comfort and compliance. It is a further advantage of embodiments of the present invention that real-time biomechanical feedback can be provided directly on the field, enhancing decision-making by avoiding delays associated with post-processing of data. Moreover, it is an advantage of embodiments of the present invention that a comprehensive biomechanical model can be developed by measuring activity across multiple muscle groups and tracking skeletal movement, thereby offering a complete picture of an athlete's physical condition.

Additionally, it is an advantage of embodiments of the present invention that contextual analysis is enabled by segmenting and classifying recorded activity into different actions, leading to more precise training and rehabilitation protocols. It is also an advantage of embodiments of the present invention that ground reaction forces can be predicted using IMU data without the need for force plates, simplifying setup and expanding usability to environments lacking specialized equipment.

Furthermore, it is an advantage of embodiments of the present invention that dynamic correction for individual athletes is possible by tailoring the biomechanical model based on their muscle activation patterns, leading to highly personalized assessments and interventions.

It is another advantage of embodiments of the present invention that preventative health management is facilitated by continuous monitoring that can identify early signs of overuse or fatigue, allowing for preventative measures before injury occurs.

It is also an advantage of embodiments of the present invention that the system can inform load management strategies, ensuring athletes train at optimal levels to maximize performance while minimizing the risk of injury.

Moreover, it is an advantage of embodiments of the present invention that versatility for future sensor integration is provided, allowing the system to evolve with advancing technology and research findings.

It is further an advantage of embodiments of the present invention that portability and ease of use are emphasized through wireless transmission of data, which is a significant improvement for field-based teams or individual athletes without access to specialized facilities.

Additionally, it is an advantage of embodiments of the present invention that data-driven rehabilitation is enabled, offering the potential to precisely measure and track return-to-play metrics, potentially reducing recovery time and improving the likelihood of a full recovery. Finally, it is an advantage of embodiments of the present invention that long-term monitoring is possible, allowing insights into long-term performance trends and health risks by comparing an athlete's current performance against baseline measurements.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG. 1 is a schematic overview of a system for performing electromyography measurements according to an embodiment of the present invention.
FIG. 2 and FIG. 3 illustrate views of a sports legging with integrated EMG sensors and IMU sensors according to embodiments of the present invention.
FIG. 4 is a block diagram of a system for performing electromyography (EMG) measurements according to embodiments of the present invention.
FIG. 5 illustrates a block diagram of a method for performing electromyography measurements according to an embodiment of the present invention.
FIG. 6 illustrates a muscle activity expectation model for walking, as can be used in embodiments of the present invention.

In the different figures, the same reference signs refer to the same or analogous elements.

### Detailed description of Illustrative Embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The following terms are provided solely to aid in the understanding of the invention.

The term "electromyography (EMG) measurements" refers to the process of recording .and analyzing the electrical activity produced by skeletal muscles through the use of electrodes placed on the skin over the muscles. Examples of EMG measurements include capturing muscle activation signals during movements like walking, jumping, or performing specific exercises. Such measurements may be performed using an "EMG unit". An example of an EMG unit is a wearable module integrated into clothing that houses multiple EMG electrodes for monitoring muscle activity.

The term "muscle activity expectation model" refers to a computational model or algorithm that predicts or identifies specific muscles engaged during a user's motion and/or activity based on sensor data. Examples include machine learning models trained on movement patterns or statistical models correlating specific activities with muscle usage.

As used herein, and unless otherwise specified, the term "wearable system" refers to a device or apparatus designed to be worn on the body, integrating electronics and sensors for data collection. Examples include smart clothing or wearable bands equipped with EMG electrodes and motion sensors. Examples include shirts, trousers, or athletic wear with embedded sensors, a bandage for a joint being a supportive wrap or brace designed to be worn around a joint, potentially integrating sensors. An example is a knee brace with built-in EMG electrodes and stretch sensors.

As used herein, and unless otherwise specified, the phrase "classifying a motion and/or activity of the user" refers to identifying and categorizing the type of movement performed by the user based on sensor data. Examples include distinguishing between running and jumping or recognizing specific gestures.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

In the first aspect, the present invention relates to a system for performing electromyography (EMG) measurements. Embodiments of the present invention may advantageously be used in the field of sports science, healthcare or ergonomics or for performing electromyography measurements for motion or activity of animals, e.g. to assess and improve performance, health, or understanding of biomechanics. Examples include monitoring athlete muscle activation during training and/or performing biomechanical screenings for detecting athletes with higher risks of injuries, assessing patient rehabilitation progress, evaluating workplace ergonomics, or studying animal locomotion patterns.

The system comprises an EMG unit for collecting electromyography data of a user, the EMG unit comprising a plurality of EMG electrodes. The system furthermore comprises one or more other sensors for capturing sensor data related to a motion and/or activity of the user. These one or more other sensors refer to additional sensing devices, apart from EMG electrodes, that capture data related to the user's motion and/or activity or optionally to other parameters of the scene. Examples include inertial measurement units (IMUs), near infrared spectroscopy sensors, heart rate sensors, GPS sensors, temperature sensors, ultrasound sensors, stretch sensors, force sensors, pressure sensors including 3D pressure sensors, and camera systems integrated into the system to provide complementary data, although this list is non-limiting and other types of sensors also may be used.

Inertial Measurement Units (IMUs) may include electronic devices that measure acceleration, rotation, and orientation using accelerometers, gyroscopes, and sometimes magnetometers. The system also comprises a sensor data processing unit programmed for processing the captured sensor data using a muscle activity expectation model to identify specific muscles used during the motion and/or activity of the user. The sensor data processing unit typically may be a computational component programmed to process captured sensor data using algorithms, models, artificial intelligence, etc.. An example is a microprocessor running software that analyzes data from the sensors and/or electrodes to determine user movements. The system furthermore comprises an EMG electrode selecting unit for selecting a subset of EMG electrodes from the plurality of EMG electrodes, the selecting being based on the identified specific muscles. This configuration allows accurate EMG measurements to be performed by selecting and using only a relevant subset of the EMG electrodes.

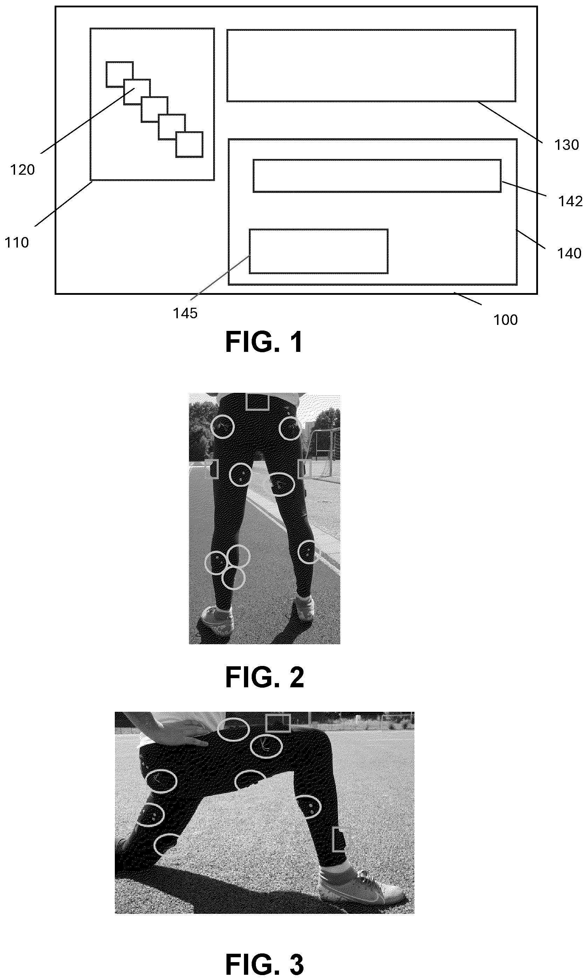

By way of illustration, embodiments of the present invention not being limited thereto, a schematic representation of a system for performing electromyography measurements is shown in FIG. 1. FIG. 1 illustrates a system 100 for performing electromyography measurements, with an EMG unit 110 comprising a plurality of EMG electrodes 120, one or more other sensors 130 and a sensor data processing unit 140. The sensor data processing unit 140 makes use of a muscle activity expectation model 142, which e.g. may be programmed on a local processor or may be implemented in a cloud computing environment. The system 100 also comprises an EMG electrode selecting unit 145 for selecting a relevant subset of the plurality of EMG electrodes 120.

By way of illustration, embodiments of the present invention not being limited thereto, an example of an EMG unit 110 including a plurality of EMG electrodes 120 as well as the one or more other sensors 130 is illustrated in FIG. 2 and FIG. 3, whereby these sensors are integrated or coupled to a garment. Embedding the sensors into stretchable garments, such as leggings, which fit tightly but comfortably against the skin, ensures unobtrusive data collection without restricting natural movement, enhancing user comfort and increasing the likelihood of compliance during activities.

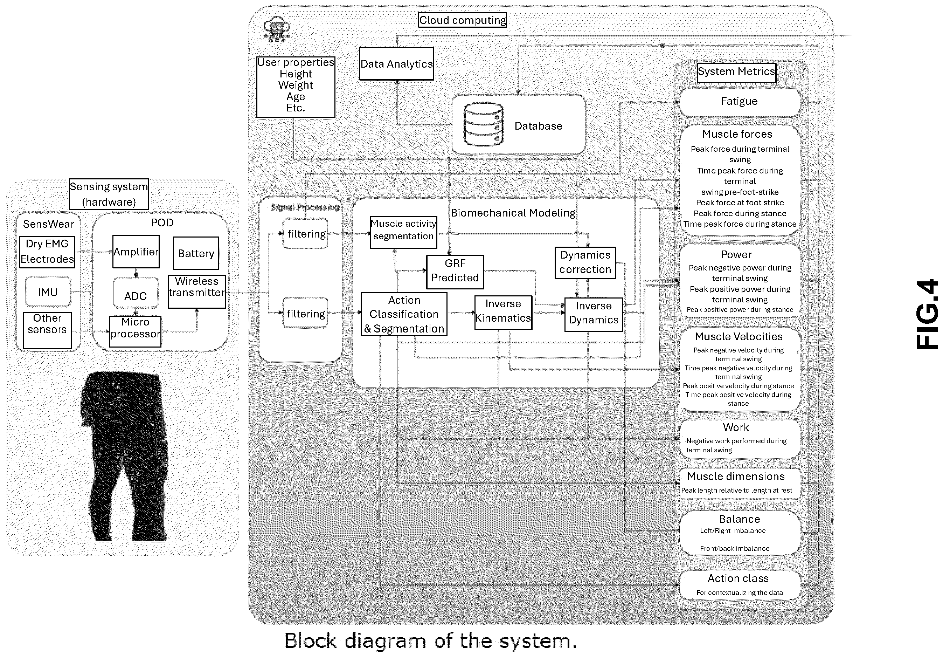

FIG. 4 illustrates an implementation of a system 100 for performing EMG measurements, integrating both a sensing system including the EMG unit 110 and one or more other sensors 130 and a processing system comprising the sensor data processing unit 140. Such a system provides comprehensive EMG measurements, showcasing the integration of hardware and software components. The sensing system is depicted on the left side of the diagram labeled "sensing system (hardware)". The sensing system in the present example is a garment, specifically a pair of leggings, with integrated EMG electrodes 120 and one or more other sensors 130. In the example given, the one or more other sensors are Inertial Measurement Units (IMUs).

In some embodiments, the system may eliminate the need for external camera systems by relying solely on integrated sensors for capturing motion and activity data. This simplifies setup and use in various environments, including on-field scenarios where cameras are impractical. Nevertheless, alternatively, also external camera systems or alike may be used.

EMG sensors may be precisely placed on specific muscles to capture detailed muscle activity, such as on three quadriceps muscles, two hamstring muscles, one gluteus muscle, and four lower leg muscles on each leg. Using a mesh of EMG sensors may allow for local variations between users and may allow - by selection of sub-sets - to adjust the measurements to individual conditions.

In the exemplary system of FIG. 4, the sensors connect to an intermediate data collection system(e.g. a POD) responsible for processing the analog signals from the sensors. Such an intermediate data collection system may contain several components arranged in a logical flow, indicating the path of data from the sensors to the wireless transmission. These components may include for example one or more of an Amplifier, an Analog-to-Digital Converter (ADC), a Microprocessor, a Battery, and a Wireless Transmitter. The Amplifier may e.g. enhance the signal strength, the ADC converts analog signals to digital form. The Microprocessor may e.g. process the digital data. The battery may power the system. The Wireless Transmitter may send the data to the data processing system.

The data processing system, shown in the box on the right hand side of FIG. 4, may include blocks for Filtering, Muscle Activity Segmentation, and Action Classification & Segmentation. The Filtering block may be the initial stage, where noise and irrelevant frequencies are removed from the signal. This is followed by Muscle Activity Segmentation, which isolates the periods of muscle activation. The Action Classification & Segmentation block then categorizes the movements and segments them for further analysis, preparing the data for biomechanical modeling.

The Cloud Computing section is in the present example responsible for advanced data analysis and modeling. It may include a Database, which stores user properties such as height and weight, and system metrics essential for personalized analysis. The Biomechanical Modeling block is central to this section, with sub-blocks for Ground Reaction Force (GRF) Prediction, Inverse Kinematics, Inverse Dynamics, and Dynamics Correction. These sub-blocks may be interconnected, demonstrating the flow of data through various modeling processes. GRF Prediction estimates the forces exerted by the ground on the user.

In this example, the system may utilize classified actions (e.g., walking, sprinting, jumping) to select action-specific prediction models for estimating ground reaction forces based on IMU data. This approach improves the accuracy of GRF predictions by tailoring them to the specific movements performed by the user. Inverse Kinematics calculates joint angles, Inverse Dynamics computes joint moments and forces, and Dynamics Correction refines these calculations for accuracy.

The system may also incorporate dynamics correction in the biomechanical model by using measurements from specific muscles. Acknowledging that different users may use varying muscle activation patterns to achieve the same movements, this personalization refines the model for each individual, enhancing the accuracy of force and moment calculations in the body.

The output from these processes feeds into the System Metrics block, which may allow to list various metrics such as Fatigue, Muscle Forces, Power, Muscle Velocities, Work, Muscle Dimensions, Balance, and Action Class, each with specific parameters related to performance and user activity. These metrics provide valuable insights into the user's biomechanical and physiological state.

The system may provide detailed analysis of skeletal movement patterns and muscle activation, identifying biomechanical inefficiencies or abnormalities. This aids in refining techniques and improving overall performance through targeted interventions.

The system also may include an output device for outputting obtained results to the user. In some embodiments, the system may include a mobile application running on devices like tablets or smartphones. This application may provide real-time visualization of biomechanical insights and system metrics, enabling interaction by medical staff, trainers, and coaches to monitor user performance, adjust training, and make informed decisions. The system of exemplary embodiments may employ strategies for generating biomechanical insights, such as creating a comprehensive dataset of metrics from multiple users to learn new insights related to injury prevention and return-to-play decisions, and establishing a baseline profile for individual users to assess readiness to return to play post-injury and identify any compensatory behaviors or deviations from baseline performance. In some embodiments, the system is capable of continuous long-term monitoring of users. This allows for comparison against baseline measurements over extended periods, providing valuable insights into performance trends, fatigue patterns, and potential health risks. In some embodiments, by monitoring muscle activity and skeletal movement in real-time, the system allows identifying early signs of overuse, fatigue, or muscle imbalances. This facilitates preventative interventions before the onset of injuries and supports tailored training adjustments to mitigate risks. In some embodiments, the system allows for informing load management strategies by assessing how intensely muscles are working during activities. This ensures users are training at optimal levels to maximize performance while minimizing injury risk, helping to avoid overtraining or undertraining by providing objective data. In some embodiments, the system provides for precise measurement and tracking of a user's rehabilitation progress post-injury, comparing rehabilitation data to baseline profiles to determine readiness to return to play. This helps in identifying any compensatory movements that need correction during recovery.

FIG. 4 illustrates how raw sensor data may be transformed into actionable insights through a combination of hardware and software components. The diagram illustrates the system's capability to provide real-time biomechanical feedback, enhancing the understanding of muscle activity and skeletal movement in athletes or users.

The system may be configured for capturing of sensor data and processing of the captured sensor data dynamically over time, and for dynamically selecting the subset of EMG electrodes based thereon. This enables dynamically switching between subsets of EMG electrodes based on the real-time output of the muscle activity expectation model, optimizing the reliability of measurements for various individual muscles at all moments in time.

As discussed, the sensor data processing unit may be programmed to classify a motion and/or activity of the user based on the captured sensor data and/or to segment the captured sensor data for identifying starting times and/or ending times of a motion and/or activity of the user. This multi-modal approach allows correlating a classified motion to specific muscle activities, enhancing the accuracy of muscle activity identification.

An advantage of a system as shown in FIG. 4 includes seamless integration into apparel, real-time data collection, comprehensive biomechanical modeling, contextual analysis, predictive ground reaction force modeling, dynamic correction for individual athletes, preventative health management, load management, versatility for future sensor integration, portability, ease of use, data-driven rehabilitation, biomechanical screening and long-term monitoring capabilities.

In one exemplary system, the system may comprise a sensing system which may be made of hardware. The sensing system may comprise senswear comprising one or more of dry EMG electrodes, one or more IMU's and other sensors. The system also may comprise a POD comprising one or more of an amplifier, a battery, a wireless transmitter, an ADC and a microprocessor. The exemplary system also may comprise a cloud computing part. The cloud computing part may comprise a data analytics component a database, a component comprising or storing user properties such as for example height, weight and age.

The cloud computing part may comprise a signal processing part with filtering components. The cloud computing part also may comprise a biomechanical modelling component having one or more of a muscle activity segmentation component, a GRF predictor, an inverse kinematics component, an inverse dynamics component, a dynamics correction component and an action classification & segmentation component.

The cloud computing part also may comprise a component for determining or providing system metrics such as fatigue, muscle forces such as peak force during terminal swing, time peak force during terminal swing pre-foot strike, peak force at foot strike, peak force during stance and time peak force during stance, power such as e.g. peak negative power during terminal swing, peak position power during terminal swing, peak positive power during stance, muscle velocities such as peak negative velocity during terminal swing, time peak negative velocity during terminal swing, peak positive velocity during stance or time peak positive velocity during stance, work such as negative work performed cduring terminal swing, muscle dimensions such as peak length relative to length at rest, balance such as left/righ imbalance and front/back imbalance, and action class for contextualizing data.

In a second aspect, the present invention relates to a method for performing electromyography (EMG) measurements. The method advantageously may be performed using a system according to the first aspect, although embodiments of the present invention are not limited thereto. The method comprises receiving sensor data related to a motion and/or activity of a user. It also comprises processing the received sensor data using a muscle activity expectation model thereby identifying specific muscles used during the motion and/or activity of the user. The method furthermore comprises selecting a subset of EMG electrodes from a plurality of EMG electrodes, the selecting being based on the identified specific muscles. Finally, the method also comprises using the subset of EMG electrodes for collecting electromyography data of a user.

According to embodiments, the method may comprise performing the capturing of sensor data and the processing of the captured sensor data dynamically over time, for dynamically selecting the subset of EMG electrodes based thereon, and dynamically collecting electromyography data using the dynamically selected subset of EMG electrodes. This dynamic approach allows the system to adapt in real-time to changes in user activity.

The method also may comprise measuring an impedance of the EMG electrodes and removing EMG electrodes with high impedance from the subset of EMG electrodes used for collecting electromyography data and/or comparing EMG electrode data with reference date for such EMG electrodes and deciding based thereon not to use the date from these EMG electrodes, i.e. remove the EMG electrodes from the actively used EMG electrodes (e.g. temporarily). This step enhances the quality of the collected data by ensuring that only electrodes with reliable contact contribute to the measurements.

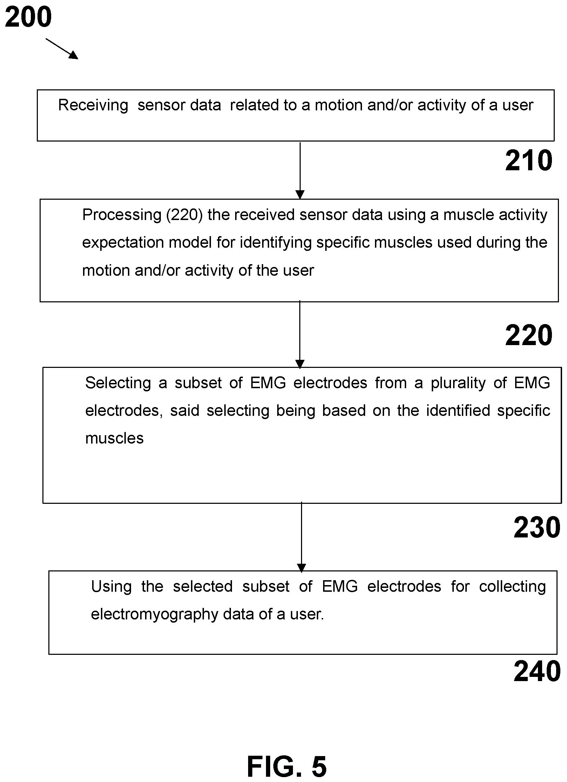

By way of illustration, FIG. 5 shows an exemplary method 200 for measuring electromyography data, including the steps of receiving 210 sensor data related to a motion and/or activity, processing 220 the received sensor data using 222 a muscle activity expectation model for identifying 224 specific muscles used during the motion and/or activity of the user, selecting 230 a subset of EMG electrodes from a plurality of EMG electrodes based on the identified specific muscles and using said subset of EMG electrodes for collecting 240 electromyography data of a user.

In a third aspect, the present invention relates to a computer program product that, when executed on a processor, assists in performing electromyography (EMG) measurements according to the method described in the second aspect, or according to the functionalities provided by a system according to the first aspect. The computer program product may be implemented as a software application or a set of instructions stored on a computer-readable medium, which when executed, facilitates the performance of electromyography measurements. Examples include software installed on a processor within the system that controls sensor data processing and electrode selection.

Further by way of illustration, a number of examples of applications are illustrated below, merely as illustration of possible applications, embodiments not being limited thereto.

In a first example an adaptive EMG electrode selection algorithm is shown. During a soccer match, the athlete wears the leggings with 20 EMG electrodes distributed across various muscle groups. As the player transitions between different activities such as jogging, sprinting, and kicking, the system dynamically selects the most relevant subset of electrodes based on the predicted muscle activity for each action. For instance, when the IMU data indicates a kicking motion, the system prioritizes the EMG electrodes on the quadriceps and hamstrings of the kicking leg. This adaptive selection ensures optimal signal quality and reduces data processing load, while allowing accurate follow up.

In a second example, the applicability in a different type of sport, e.g. swimming is shown. In swimming, the leggings are made of waterproof material with sealed EMG and IMU sensors. The system analyzes stroke efficiency by correlating upper and lower body muscle activation patterns with body rotation and kick strength. For gymnastics, additional EMG sensors are incorporated into a full-body suit to capture upper body muscle activity during complex maneuvers. The IMUs provide data on body orientation and rotation speed, allowing for detailed analysis of techniques in various apparatus routines.

Another example illustrates how such a system can help during physical therapy. The system allows comparing real-time muscle activation patterns and movement data to previously recorded baseline measurements and normative data from healthy individuals. It quantifies improvements in quadriceps strength, hamstring co-activation, and gait symmetry over time. A therapist can use this information to adjust the rehabilitation program and determine readiness for return to sport activities.

In yet another example, the system may allow for monitoring fatigue e.g. in endurance sports. For example long-distance runners can wear the leggings during marathon training. The system continuously monitors muscle activation patterns and running mechanics throughout extended training sessions. As fatigue sets in, the system detects subtle changes in muscle recruitment and gait parameters. It alerts the runner and coach when fatigue levels reach a threshold that may increase injury risk, allowing for timely adjustments to training intensity or duration.

In still another example the wearable system is adapted for use in industrial settings to assess ergonomic risks. Factory workers may wear the leggings during their shifts. The system analyzes muscle activation patterns and body postures during repetitive tasks. It identifies periods of prolonged static muscle contraction or frequent awkward postures that may lead to musculoskeletal disorders. This data informs the design of workplace interventions, such as task rotation schedules or workstation modifications, to reduce injury risk.

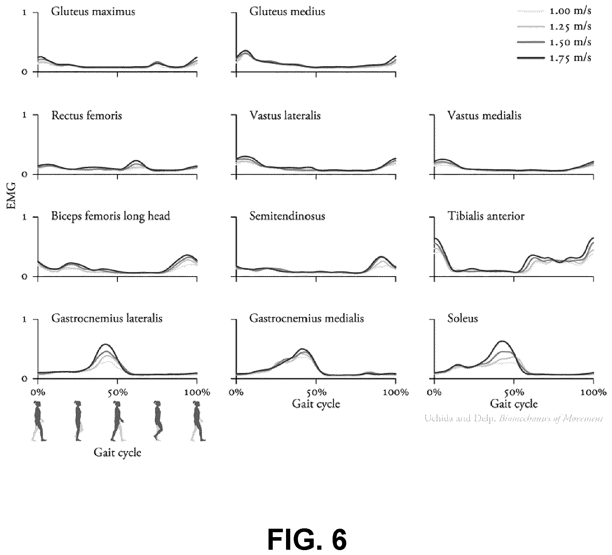

Further by way of illustration, an example of a muscle activity expectation model that can be used for an activity is illustrated with reference to FIG. 6. The muscle activity expectation model shown as example illustrates that during walking the expected muscle activation pattern is known, as indicated in FIG. 6. Similarly, expected muscle activation patterns are known or can be setup for activities such as for example running, sprinting, jumping, performing side-cutting maneuvers, squatting, kicking a ball, etc. This means that once the specific activity is detected, the specific expected activation pattern can be derived. This is helpful to further increase the accuracy of the signal acquisition in these applications.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A system (100) for performing electromyography (EMG) measurements, the system (100) comprising:
- an EMG unit (110) for collecting electromyography data of a user, the EMG unit (110) comprising a plurality of EMG electrodes (120),
- one or more other sensors (130) for capturing sensor data related to a motion and/or activity of the user,
- a sensor data processing unit (140) being programmed for processing the captured sensor data using a muscle activity expectation model (142) for identifying specific muscles used during the motion and/or activity of the user, and the system (100) being adapted for selecting a subset of EMG electrodes from the plurality of EMG electrodes (120), said selecting being based on the identified specific muscles.

2. The system (100) according to claim 1, wherein the system (100) is configured for performing said capturing sensor data and said processing of the captured sensor data dynamically over time and for dynamically selecting the subset of EMG electrodes based thereon.

3. The system (100) according to any of the previous claims, wherein the sensor data processing unit (140) is programmed for classifying a motion and/or activity of the user based on the captured sensor data and/or wherein the sensor data processing unit (140) is programmed for segmentation of the captured sensor data for identifying starting times and/or ending times of a motion and/or activity of the user.

4. The system (100) according to claim 3, wherein said identifying specific muscles is based on the classification of the motion and/or activity of the user.

5. The system (100) according to any of the previous claims, wherein the system comprises an impedance detection unit (150) configured for measuring an impedance of at least the subset of EMG electrodes (120).

6. The system (100) according to claim 5, wherein the EMG electrode selecting unit (145) furthermore is configured for removing electrodes from the subset of EMG electrodes to be used for capturing electromyography data based on one or more of at least a high impedance value and/or a degree of correlation between a measured EMG signal and a reference EMG signal.

7. The system (100) according to any of the previous claims, wherein the one or more other sensors comprise one or more of Inertial Measurement Units (IMUs), stretch sensors, force sensors, pressure sensors, camera systems.

8. The system (100) according to any of the previous claims, wherein the muscle activity expectation model is based on one or more of a machine learning technique, statistical data, time-series data, pattern recognition techniques, domain-specific knowledge, fuzzy logic, time-frequency data, to dynamically identify specific muscles used during the motions and/or activities of the user.

9. The system (100) according to any of the previous claims, the system being a wearable system.

10. The system (100) according to claim 9, wherein the wearable system is a piece of clothing.

11. The system (100) according to any of the previous claims, the system being configured for performing electromyography measurements for motion or activity of humans in the field of sports science, healthcare or ergonomics or for performing electromyography measurements for motion or activity of animals.

12. A method (200) for performing electromyography (EMG) measurements, the method (200) comprising
- receiving (210) sensor data related to a motion and/or activity of a user,
- processing (220) the received sensor data using (222) a muscle activity expectation model for identifying (224) specific muscles used during the motion and/or activity of the user,
- selecting (230) a subset of EMG electrodes from a plurality of EMG electrodes (120), said selecting (230) being based on the identified specific muscles, and
- using said subset of EMG electrodes for collecting (240) electromyography data of a user.

13. A method (200) according to claim 12, wherein the method comprises performing said capturing sensor data and said processing of the captured sensor data dynamically over time, for dynamically selecting the subset of EMG electrodes based thereon and dynamically collecting electromyography data using said dynamically selected subset of EMG electrodes.

14. A method (200) according to any of claims 12 or 13, wherein the method furthermore comprises measuring an impedance of the EMG electrodes and removing EMG electrodes with high impedance from the subset of EMG electrodes used for collecting electromyography data.

15. A computer program product for, when executed on a processor, assisting in performing electromyography (EMG) measurements, by
- receiving (210) sensor data related to a motion and/or activity of a user,
- processing (220) the received sensor data using (222) a muscle activity expectation model for identifying (224) specific muscles used during the motion and/or activity of the user, and selecting (230) a subset of EMG electrodes from a plurality of EMG electrodes (120), said selecting (230) being based on the identified specific muscles, and
- using said subset of EMG electrodes for collecting (240) electromyography data of a user.
